# EUROPÄISCHE PATENTANMELDUNG

(11) **EP 3 462 461 A1**
(43) Veröffentlichungstag der Anmeldung: **03.04.2019**
(21) Anmeldenummer: 17193766.7
(22) Anmeldetag: 28.09.2017
(51) Int. Cl.: G16H 50/50

(54) **PERSONALISIERTES PATIENTENMODELL**

(71) Anmelder: Siemens Healthcare GmbH, 91052 Erlangen (DE); Friedrich-Alexander-Universität Erlangen-Nürnberg, 91054 Erlangen (DE)
(72) Erfinder: Strobel, Norbert, 91301 Forchheim (DE); Zhong, Xia, 91054 Erlangen (DE)

(57) **Zusammenfassung**

Die Erfindung betrifft ein Verfahren für ein Berechnen eines personalisierten Patientenmodells, eine zugehörige Recheneinheit und ein zugehöriges Computerprogrammprodukt.

Das erfindungsgemäße Verfahren für ein Berechnen eines personalisierten Patientenmodells, welches ein externes Oberflächenmodell eines Patienten und ein Organmodell des Patienten aufweist, weist folgende Schritte auf:
- Erfassen von Metadaten des Patienten, wobei die Metadaten zumindest einer Metadatenkategorie zugeordnet sind,
- Ermitteln des externen Oberflächenmodells des Patienten und eines internen anatomischen Modells des Patienten, wobei das interne anatomische Modell eine Körperhöhle des Patienten aufweist, unter Verwendung der Metadaten des Patienten,
- Ermitteln des Organmodells des Patienten unter Verwendung der Metadaten des Patienten und des internen anatomischen Modells des Patienten und
- Berechnen des personalisierten Patientenmodells, wobei das externe Oberflächenmodell des Patienten und das Organmodell des Patienten kombiniert werden.

## Beschreibung

Die Erfindung betrifft ein Verfahren für ein Berechnen eines personalisierten Patientenmodells, eine zugehörige Recheneinheit und ein zugehöriges Computerprogrammprodukt.

Personalisierte Patientenmodelle, welche beispielsweise ein Oberflächenmodell eines Patienten oder ein anatomisches Modell des Patienten abbilden, werden bei einer Vielzahl an medizinischen Anwendungen verwendet.

Beispielsweise wird bei einer diagnostischen bildgebenden Untersuchung des Patienten, z.B. in einem Computertomographen, das Oberflächenmodell des Patienten mit einer Tiefenkamera erfasst und mittels des Oberflächenmodells des Patienten eine Lagerung des Patienten verbessert. In der US 2016/0306924 A1 offenbart Singh et. al ein Verfahren zum Abschätzen eines Oberflächenmodells eines Patienten mittels einer Tiefenkamera.

Typischerweise wird das personalisierte Patientenmodell bei einer Dauerbeobachtung von Dosimetriewerten, z.B. einer Hautdosis des Patienten (engl. "peak skin dose"), oder für Verbesserungen von Systemeinstellungen bei der bildgebenden Untersuchung verwendet. Ein weiteres Anwendungsfeld ist beispielsweise eine relative Positionierung eines röntgenbasierten C-Arm-Systems zu dem Patienten. Das personalisierte Patientenmodell wird insbesondere festgelegt ohne Bezug zu einer spezifischen Anatomie bzw. Morphologie des jeweiligen Patienten, sondern nur basierend auf Erfahrungswerten, z.B. aus einem größeren Patientenkollektiv, und/oder Lehrbüchern.

In manchen Anwendungsfällen werden die personalisierten Patientenmodelle gemäß einem Body-Mass-Index angepasst. Beispielsweise wird das personalisierte Patientenmodell, welches insbesondere für einen ersten Patienten berechnet wurde oder in einem Lehrbuch definiert ist, gemäß dem Body-Mass-Index eines zweiten Patienten angepasst. Üblicherweise wird ein Fettanteil des Oberflächenmodells des personalisierten Patientenmodells des ersten Patienten abhängig von dem Body-Mass-Index des zweiten Patienten skaliert. In anderen Worten wird in diesen Anwendungsfällen lediglich ein Umfang des Oberflächenmodells des personalisierten Patientenmodells des ersten Patienten verändert und kein individuelles personalisiertes Patientenmodell für den zweiten Patienten berechnet. In diesen Fällen bleiben die anatomischen Modelle oft unverändert, was eine starke Einschränkung bedeutet, da der Body-Mass-Index üblicherweise einen Einfluss auf die Anatomie und die Morphologie des Patienten hat.

Der Erfindung liegt die Aufgabe zu Grunde, ein robustes Verfahren für ein Berechnen eines personalisierten Patientenmodells mit wenigen Eingangsparametern anzugeben.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Berechnen eines personalisierten Patientenmodells, welches ein externes Oberflächenmodell eines Patienten und ein Organmodell des Patienten aufweist, weist folgende Schritte auf:
- Erfassen von Metadaten des Patienten, wobei die Metadaten zumindest einer Metadatenkategorie zugeordnet sind,
- Ermitteln des externen Oberflächenmodells des Patienten und eines internen anatomischen Modells des Patienten, wobei das interne anatomische Modell eine Körperhöhle des Patienten aufweist, unter Verwendung der Metadaten des Patienten,
- Ermitteln des Organmodells des Patienten unter Verwendung der Metadaten des Patienten und des internen anatomischen Modells des Patienten und
- Berechnen des personalisierten Patientenmodells, wobei das externe Oberflächenmodell des Patienten und das Organmodell des Patienten kombiniert werden.

Das personalisierte Patientenmodell weist insbesondere eine computerbasierte, typischerweise digitale, Kopie des Patienten auf. Das personalisierte Patientenmodell kann vorzugsweise für den Patienten individuell gemäß den Metadaten des Patienten berechnet werden. Das personalisierte Patientenmodell kann typischerweise als computerbasiertes Phantom bezeichnet werden. Das externe Oberflächenmodell weist insbesondere eine digitale Repräsentation der Oberfläche des Patienten auf. Die Oberfläche des Patienten kann beispielsweise einer Haut des Patienten entsprechen. Das Organmodell des Patienten weist insbesondere ein Organ des Patienten auf, beispielsweise ein Herz, eine Lunge, eine Milz, eine Niere oder eine Leber. Das Organmodell kann insbesondere mehrere Organe, sprich beispielsweise das Herz sowie die Leber, aufweisen. Das Organmodell weist insbesondere eine Position und/oder ein Volumen des jeweiligen Organs auf.

Das externe Oberflächenmodell, das interne anatomische Modell und das Organmodell können jeweils beispielsweise bildbasiert oder mathematisch modelliert dargestellt sein. Weitere Darstellungen oder Repräsentationen sind denkbar. Die bildbasierte, insbesondere voxelbasierte, Darstellung entspricht beispielsweise einer Aufnahme mit einer optischen Kamera oder in einem diagnostischen Bildgebungsgerät. Die mathematisch modellierte Darstellung kann einem mathematischen Modell, einem Gitternetz, Polygonen, Volumen und/oder einer Menge an Koordinaten entsprechen. Vorzugsweise kann beispielsweise die bildbasierte Darstellung des Organmodells in die mathematisch modellierte Darstellung umgerechnet werden und umgekehrt.

Die Metadaten des Patienten werden beispielsweise von einem Nutzer oder einem Arzt erfasst. Die Metadaten werden insbesondere im Rahmen einer Patientenerfassung des Patienten ohnehin erhoben oder sind beispielsweise bereits Teil einer Patientenakte des Patienten. Beispielsweise kann der Nutzer die Metadaten von dem Patienten abfragen und/oder in der Patientenakte aufrufen. Die Metadaten weisen insbesondere solche Daten auf, welche den Patienten beschreiben. Die Metadaten können beispielsweise von dem Nutzer gemessen werden. Die Metadaten können insbesondere in der Patientenakte und zusätzlich in einem geeigneten Format, beispielsweise in einem DICOM-Format (Digital Imaging and Communications in Medicine) vorliegen. Die Metadaten umfassen typischerweise nicht das personalisierte Patientenmodell.

Die zumindest eine Metadatenkategorie ermöglicht insbesondere ein Sortieren und Vergleichen der jeweils zugeordneten Metadaten. Die zumindest eine Metadatenkategorie kann Teil eines Standards, beispielsweise gemäß dem DICOM-Format gewählt sein, oder im Gegensatz quasi als Freitext wählbar sein.

Die Körperhöhle des Patienten entspricht insbesondere einem Freiraum innerhalb des Patienten. Die Körperhöhle kann alternativ oder zusätzlich das Organ, beispielsweise das Herz, aufweisen. Vorzugsweise kann die Körperhöhle mehrere Organe, beispielsweise das Herz und die Lunge aufweisen. In anderen Worten ist in diesem Fall das Organ, z.B. das Herz und die Lunge, innerhalb der Körperhöhle angeordnet. Die Körperhöhle kann in anderen Worten auch als anatomische Hülle oder konvexe Hülle bezeichnet werden.

Das interne anatomische Modell umfasst vorzugsweise eine Darstellung der Körperhöhle des Patienten. Insbesondere entspricht das interne anatomische Modell dieser Darstellung. Das interne anatomische Modell des Patienten und das Organmodell des Patienten unterscheiden sich insbesondere von dem Oberflächenmodell derart, dass sie jeweils eine Struktur, eine interne Morphologie und/oder Anatomie des Patienten aufweisen, während das Oberflächenmodell eine externe Morphologie und/oder Anatomie des Patienten beschreibt. Dabei kann die interne Morphologie und/oder Anatomie typischerweise nicht von außerhalb des Patienten mit einem menschlichen Auge erfasst werden können. Die externe Morphologie und/oder Anatomie des Patienten hingegen kann typischerweise mit dem menschlichen Auge erfasst werden.

Die Metadaten des Patienten werden vorzugsweise als Eingangsparameter für das Ermitteln des externen Oberflächenmodells des Patienten und des internen anatomischen Modells des Patienten verwendet. In diesem Fall sind vorzugsweise die Metadaten des Patienten die einzigen Parameter des Patienten, welche als die Eingangsparameter verwendet werden. Beispielsweise kann ein Modellgenerator, welcher zum Ermitteln des externen Oberflächenmodells des Patienten und des internen anatomischen Modells des Patienten ausgebildet ist und/oder trainiert ist, verwendet werden. Das externe Oberflächenmodell des Patienten und das interne anatomische Modell des Patienten sind insbesondere Ausgangsdaten des Modellgenerators, wenn die Metadaten des Patienten als die Eingangsparameter verwendet werden. Die Metadaten des Patienten sind in diesem Fall insbesondere die Eingangsparameter des Modellgenerators. Der Modellgenerator kann auf weitere Daten, welche typischerweise nicht dem Patienten zugeordnet sind, zugreifen. Die weiteren Daten können medizinische Bilddatensätze und/oder Kamerabildaufnahmen aufweisen. Die weiteren Daten werden typischerweise nicht von dem Nutzer oder dem Arzt erfasst.

Die Metadaten des Patienten und das interne anatomische Modell des Patienten werden vorzugsweise als Eingangsparameter für das Ermitteln des Organmodells verwendet. Typischerweise sind die Metadaten des Patienten und das interne anatomische Modell des Patienten dabei die einzigen Eingangsparameter, welche dem Patienten zugeordnet sind. Beispielsweise kann der Modellgenerator, welcher zum Ermitteln des externen Oberflächenmodells des Patienten und des internen anatomischen Modells des Patienten ausgebildet ist, oder ein weiterer Organmodellgenerator zum Ermitteln des Organmodells des Patienten ausgebildet und/oder trainiert sein. In diesem Fall weisen insbesondere Ausgangsdaten des Modellgenerators und/oder des Organmodellgenerators das Organmodell auf, wenn die Metadaten des Patienten und das interne anatomische Modell des Patienten als die Eingangsparameter verwendet werden. Die Metadaten des Patienten und das interne anatomische Modell des Patienten sind in diesem Fall insbesondere die Eingangsparameter des Modellgenerators und/oder des Organmodellgenerators.

Ein Trainieren des Modellgenerators und/oder des Oberflächenmodellgenerators kann in einer Trainingsphase erfolgen. Die Trainingsphase kann beispielsweise von einem Hersteller des Modellgenerators und/oder des Oberflächenmodellgenerators oder von dem Nutzer durchgeführt werden. Während der Trainingsphase wird üblicherweise ein Zusammenhang zwischen der zumindest einen Metadatenkategorie und dem externen Oberflächenmodell des Patienten, dem internen anatomischen Modells des Patienten und/oder dem Organmodell des Patienten bestimmt. In einer Anwendungsphase wird gemäß den Metaparametern des Patienten insbesondere das personalisierte Patientenmodell berechnet.

Das Kombinieren des externen Oberflächenmodells des Patienten und des Organmodells des Patienten kann beispielsweise bedeuten, dass ein Koordinatensystem des externen Oberflächenmodells und ein Koordinatensystem des Organmodells aufeinander abgestimmt werden. Das Abstimmen bedeutet ein Zusammenfügen des externen Oberflächenmodells des Patienten und des Organmodells des Patienten relativ zu dem jeweiligen Koordinatensystem. Alternativ oder zusätzlich kann das externe Oberflächenmodell des Patienten und das Organmodell des Patienten eine Relation zueinander aufweisen, wobei die Relation beim Ermitteln des externen Oberflächenmodells des Patienten, des internen anatomischen Modells des Patienten und des Organmodells des Patienten bestehen bleibt.

Das berechnete personalisierte Patientenmodell wird insbesondere bereitgestellt, z.B. auf einem Bildschirm angezeigt und/oder in einer Datenbank abgespeichert. Insbesondere wird das personalisierte Patientenmodell für einen Anwendungsfall bereitgestellt.

Eine Anwendung des personalisierten Patientenmodells kann zumindest in einem der folgenden Anwendungsfälle erfolgen:
- eine Positionierung des Patienten gemäß dem personalisierten Patientenmodell für eine bildgebende Untersuchung oder eine Therapiesitzung,
- eine Kollisionsvermeidung von einem medizinischen Bildgebungsgerät oder Therapiegerät mit dem Patienten gemäß Informationen des personalisierten Patientenmodells,
- eine Berechnung einer Dosisverteilung insbesondere auf der Haut ("peak skin dose") und/oder im Inneren des Patienten ("organ dose, equivalent patient dose") gemäß dem personalisierten Patientenmodell während der bildgebenden Untersuchung oder der Therapiesitzung,
- eine Berechnung einer Dosisverteilung in einem Untersuchungsraum, in welchem beispielsweise das medizinische Bildgebungsgerät oder Therapiegerät angeordnet ist, gemäß dem personalisierten Patientenmodell, wobei insbesondere die Dosisverteilung für den Arzt oder den Nutzer ("staff dose") berechnet wird,
- eine automatische Positionierung des medizinischen Bildgebungsgeräts oder des Therapiegeräts gemäß dem personalisierten Patientenmodell, insbesondere während der bildgebenden Untersuchung oder der Therapiesitzung, beispielsweise für eine posterior-anterior Aufnahme des Herzens des Patienten mittels eines röntgenbasierten C-Arm-Systems oder einer Angiographieanlage,
- ein Optimieren von einem Messparameter des medizinischen Bildgebungsgeräts, insbesondere von einem Röhrenstrom und/oder von einer Röhrenspannung des röntgenbasierten C-Arm-Systems, gemäß dem personalisierten Patientenmodell in Hinblick auf eine optimale Dosis und/oder Bildqualität, insbesondere in Abhängigkeit von einer Angulation des röntgenbasierten C-Arm-Systems.

Das erfindungsgemäße Verfahren für das Berechnen des personalisierten Patientenmodells bietet daher insbesondere folgende Vorteile: Das personalisierte Patientenmodell wird insbesondere lediglich basierend auf den Metadaten des Patienten als Eingangsparameter berechnet. Die mehrstufige Vorgehensweise, z.B. zunächst das Berechnen des internen anatomischen Modells und die anschließende Berechnung des Organmodells, ermöglicht üblicherweise eine besonders robuste Berechnung des personalisierten Patientenmodells. Das personalisierte Patientenmodell weist insbesondere das externe Oberflächenmodell sowie das Organmodell auf, welche lediglich unter Verwendung der Metadaten des Patienten berechnet werden. Das personalisierte Patientenmodell wird vorzugsweise ohne Verwendung einer Tiefenkamera berechnet. Das personalisierte Patientenmodell ist typischerweise besonders flexibel anwendbar, da es sowohl interne, als auch externe Informationen des Patienten umfasst. Vorzugsweise bietet das personalisierte Patientenmodell eine dreidimensionale Darstellung des gesamten Körpers des Patienten, insbesondere der Körperhöhle und des Organs.

Eine Ausführungsform sieht vor, dass die zumindest eine Metadatenkategorie mindestens einen Eintrag der folgenden Liste aufweist:
- ein Gewicht des Patienten,
- eine Größe des Patienten,
- ein Geschlecht des Patienten.

Das Gewicht des Patienten entspricht beispielsweise einem Körpergewicht und die Größe des Patienten insbesondere einer Körpergröße. Das Geschlecht des Patienten ist typischerweise männlich oder weiblich. Die Verwendung dieser Metadaten ist besonders vorteilhaft, weil die Metadaten des Patienten üblicherweise immer vorhanden und einfach zu erfassen sind. Alternativ oder zusätzlich kann die zumindest eine Metadatenkategorie eine Angulation einer Extremität des Patienten aufweisen. Die Angulation der Extremität kann beispielsweise beschreiben, dass die Extremität auf Höhe eines Kopfes des Patienten liegt oder alternativ entlang eines Oberkörpers des Patienten ausgestreckt ist. Die Angulation der Extremität ist insbesondere messbar und/oder von der bildgebenden Untersuchung bzw. einem jeweiligen Messprotokoll abhängig. Typischerweise sind bei Computertomographie-Untersuchungen des Oberkörpers des Patienten die Arme über den Kopf ausgestreckt. In einer besonders bevorzugten Ausführungsform kann die Angulation der Extremität automatisch erfasst werden, beispielsweise mittels einer optischen Kamera oder einer Tiefenkamera. Typischerweise hängt das externe Oberflächenmodell des Patienten, das interne anatomische Modell des Patienten und/oder das Organmodell des Patienten von der Angulation der Extremität ab.

Eine Ausführungsform sieht vor, dass beim Ermitteln des externen Oberflächenmodells des Patienten eine Pose des Patienten berücksichtigt wird. Die Pose des Patienten kann beispielsweise eine stehende, liegende oder sitzende Haltung beschreiben. Beispielsweise kann die Pose des Patienten derart beim Ermitteln des externen Oberflächenmodells berücksichtigt werden, dass das externe Oberflächenmodell eine stehende, liegende oder sitzende Haltung aufweist. In bestimmten Fällen kann die Pose des Patienten aufweisen, dass die Extremitäten des Patienten teilweise verschränkt und/oder ausgestreckt und/oder abgewinkelt sind. In anderen Worten ist insbesondere die Pose des Patienten von der Angulation der Extremität des Patienten abhängig. Durch die Berücksichtigung der Pose des Patienten kann vorteilhafterweise das externe Oberflächenmodell, insbesondere das personalisierte Patientenmodell, des Patienten auf die jeweilige Positionierung und/oder Haltung des Patienten abgestimmt werden.

Eine Ausführungsform sieht vor, dass das Ermitteln des externen Oberflächenmodells des Patienten eine Verwendung eines externen Deformationsmodells umfasst, wobei das externe Deformationsmodell auf die Pose des Patienten abgestimmt ist. Das externe Deformationsmodell weist insbesondere zumindest eine mathematische Transformation auf. Die Verwendung des externen Deformationsmodells umfasst insbesondere eine Anwendung des externen Deformationsmodells auf ein externes Oberflächenmodell aus einer Oberflächenmodelldatenbank, so dass das externe Oberflächenmodell an den jeweiligen Patienten angepasst wird. Hierbei umfasst die Anwendung des externen Deformationsmodells insbesondere eine Anpassung einer Pose des externen Oberflächenmodells an eine tatsächliche Pose des Patienten. Beispielsweise kann ein stehendes externes Oberflächenmodell in ein liegendes externes Oberflächenmodell mittels der Verwendung des externen Deformationsmodells transformiert werden. Das externe Deformationsmodell ermöglicht insbesondere die Deformation des externen Oberflächenmodells gemäß der Haltung und/oder einer Lagerung des Patienten. Beispielsweise kann mittels des externen Deformationsmodells das personalisierte Patientenmodell, insbesondere das Oberflächenmodell, eines auf einer Patientenliege auf dem Bauch liegend angeordneten Patienten berechnet werden. In einem weiteren Beispiel ermöglicht das externe Deformationsmodell das Ermitteln des Oberflächenmodells des Patienten, wobei das Oberflächenmodell beispielsweise eine sitzende Haltung mit ausgestreckten Extremitäten aufweist. Das externe Deformationsmodell kann beispielsweise einen durchgestreckten Arm abwinkeln und umgekehrt.

Eine Ausführungsform sieht vor, dass das externe Deformationsmodell auf physikalische Kräfte abgestimmt ist und wobei die physikalischen Kräfte die Pose des Patienten beeinflussen. Die physikalischen Kräfte beschreiben beispielsweise eine Gravitationskraft, ein lokal herrschendes Schwerefeld und/oder auf den Patienten wirkende Trägheitswirkungen. Typischerweise hängt das externe Deformationsmodell von einer Ausrichtung der Pose des Patienten relativ zu einem Richtungsvektor der physikalischen Kräfte ab. Die physikalischen Kräfte können beispielsweise durch eine Form der Patientenliege vorgegeben sein, wenn der Patient beispielsweise breiter als eine Auflagefläche der Patientenliege ist. Die physikalischen Kräfte sind insbesondere ungleich 0, wenn das externe Deformationsmodell die Transformation von der stehenden Haltung in die sitzende Haltung beschreibt. Die physikalischen Kräfte beeinflussen die Pose insbesondere derart, dass sich beispielsweise ein Bauch eines adipösen Patienten sich in der stehenden oder liegenden Pose unterschiedlich darstellt.

Eine Ausführungsform sieht vor, dass das Ermitteln des externen Oberflächenmodells des Patienten und des internen anatomischen Modells des Patienten unter Verwendung einer Oberflächenmodelldatenbank, welche externe Oberflächenmodelle aufweist, wobei jedem externem Oberflächenmodell oberflächenmodellbezogene Metadaten zugeordnet sind, und unter Verwendung einer medizinischen Bilddatenbank, welche medizinische Bilddatensätze aufweist, wobei jedem medizinischen Bilddatensatz medizinische Metadaten zugeordnet sind, erfolgt, wobei die oberflächenmodellbezogenen Metadaten und die medizinischen Metadaten der zumindest einen Metadatenkategorie zugeordnet sind.

Die externen Oberflächenmodelle der Oberflächenmodelldatenbank sind insbesondere während einer äußeren Vermessung mehrerer Trainingspersonen generiert worden. Typischerweise werden die mehreren Trainingspersonen in stehender oder sitzender Pose mittels einer Tiefenkamera oder optischer Kameras für eine dreidimensionale Abbildung der mehreren Trainingspersonen erfasst. Zusätzlich werden üblicherweise den dreidimensionalen Abbildungen die oberflächenmodellbezogenen Metadaten zugeordnet, wobei die oberflächenmodellbezogenen Metadaten die jeweilige Trainingsperson beschreiben. Von den dreidimensionalen Abbildungen der mehreren Trainingspersonen werden insbesondere die externen Oberflächenmodelle berechnet, wobei die oberflächenmodellbezogenen Metadaten entsprechend zugeordnet sind. Die Oberflächenmodelldatenbank kann beispielsweise von einer Kleidungsindustrie gebildet worden sein. Alternativ oder zusätzlich kann die Oberflächenmodelldatenbank externe Oberflächenmodelle aufweisen, welche beispielsweise bei Strahlenschutzuntersuchungen erfasst worden sind. Die Strahlenschutzuntersuchungen werden üblicherweise in stehender Pose durchgeführt.

Die medizinischen Bilddatensätze der medizinischen Bilddatenbank sind insbesondere während bildgebender Untersuchungen von Trainingspersonen mittels eines medizinischen Bildgebungsgeräts generiert worden. Typischerweise ist die medizinische Bilddatenbank dort verfügbar, wo die bildgebenden Untersuchungen durchgeführt oder die bei den bildgebenden Untersuchungen erfassten medizinischen Bilddatensätze verarbeitet werden. Die medizinische Bilddatenbank kann beispielsweise einem Radiologieinformationssystem (RIS) oder einem Bildarchivierungssystem (PACS, Picture Archiving and Communication System) entsprechen. Abhängig von der bildgebenden Untersuchung weist der medizinische Bilddatensatz eine entsprechende Pose der jeweiligen untersuchten Trainingsperson auf. Typischerweise weisen die medizinischen Bilddatensätze einer Computertomographie- oder Magnetresonanztomographie-Untersuchung die liegende Haltung bzw. Pose auf, während beispielsweise die medizinischen Bilddatensätze einer röntgenbasierten Mammographie-Untersuchung die stehende Pose aufweisen können. Die medizinischen Bilddatensätze können insbesondere jedem medizinischen Bildgebungsgeräts entstammen. Die medizinischen Bilddatensätze bilden vorzugsweise eine Anatomie, eine Struktur und/oder eine Morphologie der untersuchten Trainingsperson ab. Das interne anatomische Modell basiert insbesondere auf einer Abbildung der Struktur, der Morphologie und/oder der Anatomie in den medizinischen Bilddatensätzen. Von den medizinischen Bilddatensätzen können insbesondere die internen anatomischen Modelle berechnet werden. Typischerweise ist jedem medizinischen Bilddatensatz des jeweiligen medizinischen Bildgebungsgeräts die medizinischen Metadaten der jeweiligen untersuchten Trainingsperson zugeordnet. Üblicherweise wird das DICOM-Format für die medizinischen Bilddatensätze verwendet, um eine jeweilige Bildinformation mit den entsprechenden medizinischen Metadaten zu verknüpfen. Typischerweise sind mehr externe Oberflächenmodelle als medizinische Bilddatensätze verfügbar.

Die medizinischen Bilddatensätze verfügen üblicherweise nur in seltenen Fällen über eine Abbildung des gesamten Körpers der Trainingsperson. Im Gegensatz dazu weisen die externen Oberflächenmodelle typischerweise eine Abbildung des gesamten Körpers auf. Die medizinischen Datensätze weisen typischerweise einen Abschnitt des gesamten Körpers auf, beispielsweise Beine der Trainingsperson und/oder einen Thorax der Trainingsperson. Von den medizinischen Bilddatensätzen kann vorzugsweise ein externes medizinisches Oberflächenmodell zumindest abschnittsweise berechnet werden, insbesondere wenn die medizinischen Bilddatensätze eine Oberfläche, vorzugsweise eine Haut, aufweisen.

Dass die Metadaten des Patienten, die oberflächenmodellbezogenen Metadaten und die medizinischen Metadaten der zumindest einen Metadatenkategorie zugeordnet sind, bedeutet insbesondere, dass die Metadaten des Patienten, die oberflächenmodellbezogenen Metadaten und die medizinischen Metadaten vorzugsweise ein gemeinsames Merkmal und/oder eine Gemeinsamkeit aufweisen. Die zumindest eine Metadatenkategorie ist daher insbesondere ein vergleichbarer Parameter, wobei die Metadaten des Patienten, die oberflächenmodellbezogenen Metadaten und die medizinischen Metadaten den vergleichbaren Parameter aufweisen. Es ist denkbar, dass die Metadaten des Patienten weitere Metadatenkategorien aufweisen, welche die oberflächenmodellbezogenen Metadaten und/oder die medizinischen Metadaten typischerweise jeweils nicht aufweisen und umgekehrt. Die zumindest eine Metadatenkategorie bildet insbesondere eine Verbindung zwischen den medizinischen Bilddatensätzen und den externen Oberflächenmodellen.

Eine Ausführungsform sieht vor, dass das Ermitteln des externen Oberflächenmodells des Patienten unter Verwendung der Oberflächenmodelldatenbank und der medizinischen Bilddatenbank erfolgt und wobei das Ermitteln des internen anatomischen Modells des Patienten ausschließlich unter Verwendung der medizinischen Bilddatenbank erfolgt. Vorzugsweise weisen die medizinischen Bilddatensätze der medizinischen Bilddatenbank die Pose oder zumindest eine ähnliche Pose im Vergleich zu den externen Oberflächenmodellen der Oberflächenmodelldatenbank auf. Insbesondere wenn die medizinischen Bilddatensätze und die externen Oberflächenmodelle dieselbe Pose aufweisen, kann das Anwenden des externen Deformationsmodells überflüssig sein. In diesem Fall wird vorzugsweise lediglich das externe Oberflächenmodell ermittelt und typischerweise das externe Deformationsmodell weder auf das externe Oberflächenmodell noch auf das interne anatomische Modell angewendet. In anderen Worten erfolgt insbesondere das Berechnen des externen Deformationsmodells unter Verwendung der Oberflächenmodelldatenbank und der medizinischen Bilddatenbank.

Eine Ausführungsform sieht vor, dass das Ermitteln des internen anatomischen Modells des Patienten unter Verwendung der medizinischen Bilddatenbank erfolgt, welche die medizinischen Bilddatensätze aufweist, wobei jedem medizinischen Bilddatensatz die medizinischen Metadaten zugeordnet sind, wobei die medizinischen Metadaten der zumindest einen Metadatenkategorie zugeordnet sind, und ein Auswählen zumindest eines medizinischen Bilddatensatzes aus den medizinischen Bilddatensätzen der medizinischen Bilddatenbank gemäß den Metadaten des Patienten und ein Ermitteln der Körperhöhle des internen anatomischen Modells des Patienten unter Verwendung des zumindest einen medizinischen Bilddatensatzes umfasst. Das Auswählen des zumindest einen medizinischen Bilddatensatzes kann ein Lernen eines ersten Zusammenhangs zwischen der zumindest einen Metadatenkategorie und den medizinischen Bilddatensätzen, welchen die medizinischen Metadaten zugeordnet sind, umfassen. Gemäß dem ersten Zusammenhang wird vorzugsweise der zumindest eine medizinische Bilddatensatz gemäß den Metadaten des Patienten ausgewählt. Der zumindest eine medizinische Bilddatensatz wird vorteilhafterweise möglichst passend zum Patienten ausgewählt, da die Metadaten des Patienten als die Eingangsparameter für die Auswahl verwendet werden. Der zumindest eine medizinische Bilddatensatz kann möglicherweise aus mehreren medizinischen Bilddatensätzen gemäß den Metadaten des Patienten berechnet sein, beispielsweise mittels einer Mittelwertbildung oder ähnlicher mathematischer Verfahren.

Das Lernen des ersten Zusammenhangs kann eine erste Regressionsanalyse aufweisen. Allgemein gesprochen ist die Regressionsanalyse ein statistisches Verfahren, um Zusammenhänge zwischen einer abhängigen und einer unabhängigen Variable zu modellieren. Der Zusammenhang zwischen den Variablen ist üblicherweise in Regressionsvorschriften abgebildet. Die Regressionsanalyse löst typischerweise ein Regressionsproblem. Für die Lösung des Regressionsproblems werden üblicherweise verschiedene Verfahren verwendet, insbesondere eine lineare oder multi-lineare Regression, eine Support Vector Machine (SVM), eine Kernel-SVM, und/oder Deep-Learning-Verfahren mittels künstlicher neuronaler Netze. Mittels der Regressionsanalyse können vorzugsweise unter Verwendung von Eingangsparametern Ausgangsdaten ermittelt oder abgeschätzt werden. Derart kann auch das Ermitteln des externen Oberflächenmodells des Patienten und des internen anatomischen Modells des Patienten ein Anwenden einer Regressionsanalyse aufweisen, wobei die Metadaten des Patienten als die Eingangsparameter verwendet werden. Das Ermitteln des Organmodells des Patienten weist insbesondere ein weiteres Anwenden der Regressionsanalyse auf, wobei die Metadaten des Patienten als die Eingangsparameter verwendet werden. Das Ermitteln entspricht in diesen Fällen insbesondere einem Bereitstellen des externen Oberflächenmodells des Patienten und des internen anatomischen Modells des Patienten sowie des Organmodells des Patienten als Ausgangsdaten der jeweiligen Regressionsanalyse.

Eine Ausführungsform sieht vor, dass das Ermitteln des externen Oberflächenmodells des Patienten unter Verwendung der Oberflächenmodelldatenbank erfolgt, welche die externen Oberflächenmodelle aufweist, wobei jedem externem Oberflächenmodell die oberflächenmodellbezogene Metadaten zugeordnet sind, wobei die oberflächenmodellbezogenen Metadaten der zumindest einen Metadatenkategorie zugeordnet sind, und folgende Schritte umfasst:
- Auswählen eines abgeschätzten externen Oberflächenmodells aus den externen Oberflächenmodellen der Oberflächenmodelldatenbank gemäß den Metadaten des Patienten,
- Berechnen eines externen Deformationsmodells unter Verwendung des abgeschätzten externen Oberflächenmodells und
- Anwenden des externen Deformationsmodells auf das abgeschätzte externe Oberflächenmodell, wobei das externe Oberflächenmodell des Patienten erzeugt wird.

Das Auswählen des abgeschätzten externen Oberflächenmodells kann ein Lernen eines zweiten Zusammenhangs zwischen der zumindest einen Metadatenkategorie und den externen Oberflächenmodellen, welchen die oberflächenmodellbezogenen Metadaten zugeordnet sind, umfassen. Üblicherweise weist das Lernen des zweiten Zusammenhangs eine zweite Regressionsanalyse auf. Gemäß dem zweiten Zusammenhang wird vorzugsweise das abgeschätzte externe Oberflächenmodell gemäß den Metadaten des Patienten ausgewählt. Das abgeschätzte externe Oberflächenmodell wird vorteilhafterweise möglichst passend zum Patienten ausgewählt, da die Metadaten des Patienten als die Eingangsparameter für die Auswahl verwendet werden. Das abgeschätzte externe Oberflächenmodell kann insbesondere möglicherweise aus mehreren ähnlichen externen Oberflächenmodellen gemäß den Metadaten des Patienten berechnet sein, beispielsweise mittels der Mittelwertbildung oder ähnlicher mathematischer Verfahren.

Basierend auf dem abgeschätzten externen Oberflächenmodell wird das externe Deformationsmodell insbesondere auf die Pose des Patienten abgestimmt. Das abgeschätzte externe Oberflächenmodell ist insbesondere ein erster Eingangsparameter für das Berechnen des externen Deformationsmodells. Beim Anwenden des externen Deformationsmodells auf das abgeschätzte externe Oberflächenmodell wird das abgeschätzte externe Oberflächenmodell beispielsweise gemäß der Pose des Patienten transformiert. Das abgeschätzte externe Oberflächenmodell weist beispielsweise eine stehende Pose auf, während das externe Oberflächenmodell des Patienten insbesondere eine liegende Pose aufweist. Das abgeschätzte externe Oberflächenmodell und das externe Oberflächenmodell des Patienten unterscheiden sich insbesondere durch das Anwenden des externen Deformationsmodells.

Alternativ oder zusätzlich kann das externe Deformationsmodell auf das interne anatomische Modell des Patienten angewendet werden. In diesem Fall werden gemäß dem externen Oberflächenmodell beispielsweise die Körperhöhle, insbesondere eine Position und/oder ein Volumen der Körperhöhle, angepasst. Die Relation zwischen dem externen Oberflächenmodell des Patienten und dem Organmodell des Patienten wird entsprechend angepasst. Dies ist insbesondere vorteilhaft, wenn das externe Deformationsmodell von der Angulation der Extremität des Patienten abhängt. Beispielsweise verschiebt sich die Position der Lunge relativ zu der Haut, wenn das externe Deformationsmodell eine Transformation von über den Kopf ausgestreckten Armen des Patienten zu auf dem Bauch überkreuzt liegenden Armen des Patienten beschreibt.

Eine Ausführungsform sieht vor, dass das Berechnen des externen Deformationsmodells unter Verwendung der medizinischen Bilddatenbank erfolgt, welche die medizinischen Bilddatensätze aufweist, wobei jedem medizinischen Bilddatensatz die medizinischen Metadaten zugeordnet sind, wobei die medizinischen Metadaten der zumindest einen Metadatenkategorie zugeordnet sind, und folgende Schritte umfasst:
- Auswählen des zumindest einen medizinischen Bilddatensatzes aus den medizinischen Bilddatensätzen der medizinischen Bilddatenbank gemäß den Metadaten des Patienten und
- Registrieren des abgeschätzten externen Oberflächenmodells und des zumindest einen medizinischen Bilddatensatzes.

Das Auswählen des zumindest einen medizinischen Bilddatensatzes kann beispielsweise gemäß dem ersten Zusammenhang bzw. unter Verwendung der ersten Regressionsanalyse erfolgen. Grundsätzlich ist denkbar, dass eine weitere Regressionsanalyse für ein Lernen eines weiteren Zusammenhangs zwischen der zumindest einen Metadatenkategorie und den medizinischen Bilddatensätzen, welche insbesondere die medizinischen Metadaten aufweisen, erfolgt. Die Metadaten des Patienten sind in diesem Fall Eingangsparameter für die weitere Regressionsanalyse und Ausgangsdaten weisen insbesondere den zumindest einen medizinischen Bilddatensatz auf. Üblicherweise erfolgt das Registrieren gemäß einer rigiden Registrierung. Insbesondere gemäß der rigiden Registrierung kann das externe Deformationsmodell berechnet werden.

Eine Ausführungsform sieht vor, dass vor dem Registrieren des abgeschätzten externen Deformationsmodells der zumindest eine medizinischen Bilddatensatz gemäß einer ersten Segmentierungsvorschrift segmentiert wird, und/oder dass die Körperhöhle des internen anatomischen Modells des Patienten durch ein Segmentieren des zumindest einen medizinischen Bilddatensatzes gemäß einer zweiten Segmentierungsvorschrift ermittelt wird.

Die erste Segmentierungsvorschrift kann insbesondere gemäß einer Haut und/oder einer Oberfläche des jeweiligen Patienten ausgebildet sein. In anderen Worten wird beim Segmentieren gemäß der ersten Segmentierungsvorschrift insbesondere die Haut in dem zumindest einen medizinischen Bilddatensatz ermittelt. Die Haut ist insbesondere in der Morphologie, in der Struktur und/oder der Anatomie in den medizinischen Bilddatensätzen abgebildet. Der erste segmentierte zumindest eine medizinische Bilddatensatz entspricht vorzugsweise dem externen medizinischen Oberflächenmodell. Das externe medizinische Oberflächenmodell wird vorzugsweise anstelle des zumindest einen medizinischen Bilddatensatzes beim Registrieren mit dem abgeschätzten externen Oberflächenmodell verwendet. Das externe medizinische Oberflächenmodell ist insbesondere ein zweiter Eingangsparameter für das Berechnen des externen Deformationsmodells.

Die zweite Segmentierungsvorschrift ermöglicht insbesondere das Ermitteln der anatomischen Hülle oder der konvexen Hülle, beispielsweise um ein in dem zumindest einen medizinischen Bilddatensatz abgebildeten Organ. Die Morphologie, die Struktur und/oder die Anatomie in den medizinischen Bilddatensätzen weisen insbesondere die anatomische Hülle, typischerweise die Körperhöhle, und insbesondere das von der anatomischen Hülle umfasste Organ auf. Die Körperhöhle entspricht insbesondere der anatomischen Hülle bzw. der konvexen Hülle. Zwei typische Beispiele für die anatomische Hülle sind insbesondere eine Brusthöhle (Cavitas thoracis) und eine Bauchhöhle (Cavitas abdominalis).

Eine Ausführungsform sieht vor, dass das interne anatomische Modell zusätzlich zu der Körperhöhle eine weitere Körperhöhle aufweist, wobei die Körperhöhle einen Thorax des Patienten und die weitere Körperhöhle einen Abdomen des Patienten aufweist und wobei beim Ermitteln des Organmodells des Patienten ein Abstand zwischen der Körperhöhle Thorax und der weiteren Körperhöhle Abdomen minimiert wird. Die Körperhöhle Thorax entspricht insbesondere der Brusthöhle und die weitere Körperhöhle Abdomen entspricht insbesondere der Bauchhöhle. Das Organ kann insbesondere innerhalb der Körperhöhle oder der weiteren Körperhöhle angeordnet sein. Die mehreren Organe können jeweils insbesondere innerhalb der Körperhöhle oder der weiteren Körperhöhle angeordnet sein. Beispielsweise kann die Körperhöhle Thorax das Herz und/oder die Lunge des Patienten aufweisen, während die weitere Körperhöhle Abdomen die Leber des Patienten aufweist. In anderen Worten bildet die Körperhöhle Thorax eine erste konvexe Hülle um das Herz bzw. die Lunge und die weitere Körperhöhle Abdomen bildet eine zweite konvexe Hülle um die Leber. Typischerweise ist eine Schnittmenge zwischen der Körperhöhle und der weiteren Körperhöhle leer. In diesem Fall kann insbesondere ein Abstand zwischen dem Herz und der Leber oder zwischen der Lunge und der Leber minimiert werden. Eine Vorgabe für das Minimieren kann beispielsweise medizinische Standardliteratur aufweisen. Das Organmodell kann die Körperhöhle und die weitere Körperhöhle, insbesondere die Körperhöhle Thorax und die weitere Körperhöhle Abdomen, aufweisen. Beispielsweise kann der Abstand zwischen der Körperhöhle Thorax und der Körperhöhle Abdomen anstelle beim Ermitteln des internen anatomischen Modells beim Ermitteln des Organmodells minimiert werden.

Eine Ausführungsform sieht vor, dass das Ermitteln des Organmodells des Patienten unter Verwendung der medizinischen Bilddatenbank, welche die medizinischen Bilddatensätze aufweist, wobei jedem medizinischen Bilddatensatz die medizinische Metadaten zugeordnet sind, erfolgt, wobei die medizinischen Metadaten der zumindest einen Metadatenkategorie zugeordnet sind. Typischerweise werden für das Ermitteln des Organmodells des Patienten ausschließlich die medizinische Bilddatenbank und die Metadaten des Patienten verwendet. Üblicherweise kann die medizinische Bilddatenbank für das Ermitteln des Organmodells des Patienten insbesondere der medizinischen Bilddatenbank für das Ermitteln des Oberflächenmodells des Patienten und des internen anatomischen Modells des Patienten entsprechen. In anderen Worten kann die medizinische Bilddatenbank für das Ermitteln des Organmodells des Patienten sowie für das Ermitteln des Oberflächenmodells des Patienten und des internen anatomischen Modells des Patienten verwendet werden.

Eine Ausführungsform sieht vor, dass das Ermitteln des Organmodells folgende Schritte umfasst:
- Auswählen des zumindest einen medizinischen Bilddatensatzes aus den medizinischen Bilddatensätzen der medizinischen Bilddatenbank gemäß den Metadaten des Patienten,
- Segmentieren des zumindest einen medizinischen Bilddatensatzes gemäß einer dritten Segmentierungsvorschrift,
- Berechnen zumindest eines internen Deformationsmodells unter Verwendung des internen anatomischen Modells des Patienten und des segmentierten zumindest einen medizinischen Bilddatensatzes,
- Anwenden des zumindest einen internen Deformationsmodells auf den segmentierten zumindest einen medizinischen Bilddatensatz und
- Klassifizieren des internen anatomischen Modells des Patienten gemäß dem deformierten zumindest einen medizinischen Bilddatensatz, wobei das Organmodell erzeugt wird.

Das Auswählen des zumindest einen medizinischen Bilddatensatzes kann vorzugsweise gemäß dem ersten Zusammenhang bzw. unter Verwendung der ersten Regressionsanalyse erfolgen. Grundsätzlich es denkbar, dass eine weitere Regressionsanalyse für ein Lernen eines weiteren Zusammenhangs zwischen der zumindest einen Metadatenkategorie und den medizinischen Bilddatensätzen, welche insbesondere die medizinischen Metadaten aufweisen, erfolgt. In diesem Fall kann der zumindest eine medizinische Bilddatensatz unter Verwendung der weiteren Regressionsanalyse ausgewählt werden.

Die dritte Segmentierungsvorschrift ermöglicht insbesondere das Ermitteln der anatomischen Hülle oder der konvexen Hülle, insbesondere um das Organ in dem zumindest einen medizinischen Bilddatensatz. Vorzugsweise weist der segmentierte zumindest eine medizinische Bilddatensatz die anatomische Hülle um das Organ und das Organ selbst auf. Insbesondere wenn zusätzlich zu dem Organ ein weiteres Organ in dem zumindest einen medizinischen Bilddatensatz abgebildet ist und die anatomische Hülle das Organ und das weitere Organ aufweist, weist der segmentierte zumindest eine medizinische Bilddatensatz die anatomische Hülle um das Organ und das weitere Organ sowie das Organ und das weitere Organ auf. Abhängig von einer Position des Organs und des weiteren Organs zueinander, kann die anatomische Hülle das Organ, das weitere Organ und einen Füllraum aufweisen, wobei eine Summe aus dem Organ, dem weiteren Organ und dem Füllraum ein Volumen der anatomischen Hülle ergibt. Die anatomische Hülle, die konvexe Hülle und die Körperhöhle entsprechen sich üblicherweise gegenseitig. Typischerweise kann die dritte Segmentierungsvorschrift der zweiten Segmentierungsvorschrift entsprechen.

Das Berechnen des zumindest einen internen Deformationsmodells weist insbesondere eine nicht-rigide Registrierung des internen anatomischen Modells des Patienten und des segmentierten zumindest einen medizinischen Bilddatensatzes auf. Beispielsweise wird vor der nicht-rigiden Registrierung die mathematisch modellierte Darstellung des internen anatomischen Modells des Patienten und die mathematisch modellierte Darstellung des zumindest einen medizinischen Bilddatensatzes in die jeweilige bildbasierte Darstellung umgerechnet. Typischerweise wird die rigide und/oder die nicht-rigide Registrierung bildbasiert durchgeführt. Die rigide Registrierung umfasst insbesondere eine Rotation und Translation, während die nicht-rigide Registrierung insbesondere eine elastische Transformation aufweist. Beispielsweise können für die rigide und/oder die nicht-rigide Registrierung Landmarken, insbesondere in den zumindest einen medizinischen Bilddatensätzen, gesetzt werden, wobei die Landmarken die Registrierung vereinfachen können.

Mittels des Anwendens des zumindest einen internen Deformationsmodells wird insbesondere der zumindest eine medizinische Bilddatensatz verformt. Wenn der zumindest eine medizinische Bilddatensatz die Körperhöhle und beispielsweise das Organ innerhalb der Körperhöhle aufweist, werden vorzugsweise die Körperhöhle und das Organ gemäß dem zumindest einen internen Deformationsmodell verformt.

Das interne anatomische Modell weist typischerweise vor dem Klassifizieren das Organ nicht auf, sondern ausschließlich die Körperhöhle, worin das Organ eingebettet werden soll. Der deformierte und insbesondere segmentierte zumindest eine Bilddatensatz weist vorzugsweise das Organ, beispielsweise zusätzlich das weitere Organ, sowie die Körperhöhle auf. Üblicherweise ist nach dem Anwenden des zumindest einen internen Deformationsmodells die Körperhöhle des internen anatomischen Modells und die Körperhöhle, insbesondere die anatomische Hülle um das Organ und das weitere Organ, in dem zumindest einen medizinischen Bilddatensatz deckungsgleich. Vorzugsweise kann das Klassifizieren des internen anatomischen Modells des Patienten ein voxelbasiertes Überprüfen umfassen, ob das jeweilige Voxel in dem zumindest einen medizinischen Bilddatensatz das Organ aufweist. Falls das jeweilige Voxel das Organ aufweist, wird ein entsprechendes Voxel des internen anatomischen Modells des Patienten ebenfalls als das Organ klassifiziert. Nach dem Klassifizieren weist vorzugsweise das interne anatomische Modell des Patienten das Organ auf, wenn der deformierte zumindest eine medizinische Bilddatensatz ebenfalls das Organ aufweist. Dadurch wird insbesondere das Organmodell erzeugt.

Die erfindungsgemäße Recheneinheit für das Berechnen des personalisierten Patientenmodells weist ein Rechenmodul auf. Die Recheneinheit ist insbesondere zum Berechnen des personalisierten Patientenmodells ausgebildet. Das Rechenmodul weist insbesondere einen Prozessor und einen Arbeitsspeicher auf, wobei der Prozessor vorzugsweise die Verfahrensschritte zum Berechnen des personalisierten Patientenmodells ausführen kann. Die Recheneinheit kann zusätzlich ein Eingabegerät und den Bildschirm aufweisen, wobei beispielsweise der Nutzer die Metadaten des Patienten mittels des Eingabegeräts erfassen und insbesondere auf dem Bildschirm anzeigen kann. Vorzugsweise kann der Bildschirm das personalisierte Patientenmodell anzeigen und/oder auf einer Festplatte des Recheneinheit abspeichern. Die Recheneinheit kann beispielsweise über geeignete Schnittstelle zu einem Computernetzwerk oder zu dem medizinischen Bildgebungsgerät oder zu einer externen Speichereinheit das personalisierte Patientenmodell und/oder die Metadaten des Patienten übertragen und/oder von dort abrufen. Die Recheneinheit ist vorzugsweise mit dem Computernetzwerk, insbesondere der Oberflächenmodelldatenbank und/oder der medizinischen Bilddatenbank verbunden. Alternativ oder zusätzlich kann die Recheneinheit oder die an die Recheneinheit angeschlossene externe Speichereinheit die Oberflächenmodelldatenbank und/oder die medizinische Bilddatenbank aufweisen. Die Recheneinheit kann insbesondere den Modellgenerator und/oder den Organmodellgenerator aufweisen.

Das erfindungsgemäße Computerprogrammprodukt, welches direkt in einen Speicher einer programmierbaren Recheneinheit ladbar ist, mit Programmcodemitteln, um ein Verfahren nach einem der Ansprüche 1 bis 14 auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist vorzugsweise so konfiguriert, dass es mittels der Recheneinheit die erfindungsgemäßen Verfahrensschritte ausführen kann. Das Computerprogrammprodukt ist beispielsweise auf einem computerlesbaren Medium gespeichert oder in dem Computernetzwerk oder auf einem Server hinterlegt, von wo es in den Prozessor der Recheneinheit geladen werden kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, welche insbesondere Programmcodemitteln entsprechen, gespeichert sind. Wenn diese Steuerinformationen von dem Datenträger gelesen und in der Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen. Das Computerprogrammprodukt kann insbesondere den Modellgenerator und/oder den Organmodellgenerator aufweisen.

Weitere Merkmale, Vorteile oder alternative Ausführungsformen des erfindungsgemäßen Verfahrens zum Berechnen des personalisierten Patientenmodells und/oder der zugehörigen Recheneinheit und/oder des zugehörigen Computerprogrammprodukts können ebenso auch auf die jeweiligen anderen beanspruchten Gegenstände übertragen werden und umgekehrt.

Im Folgenden wird die Erfindung anhand der in den Figuren dargestellten Ausführungsbeispiele näher beschrieben und erläutert.

Es zeigen:
Fig. 1 ein Verfahren in einem ersten Ausführungsbeispiel,
Fig. 2 ein Verfahren in einem zweiten Ausführungsbeispiel,
Fig. 3 ein Verfahren in einem dritten Ausführungsbeispiel,
Fig. 4 ein Verfahren in einem vierten Ausführungsbeispiel und
Fig. 5 ein personalisiertes Patientenmodell.

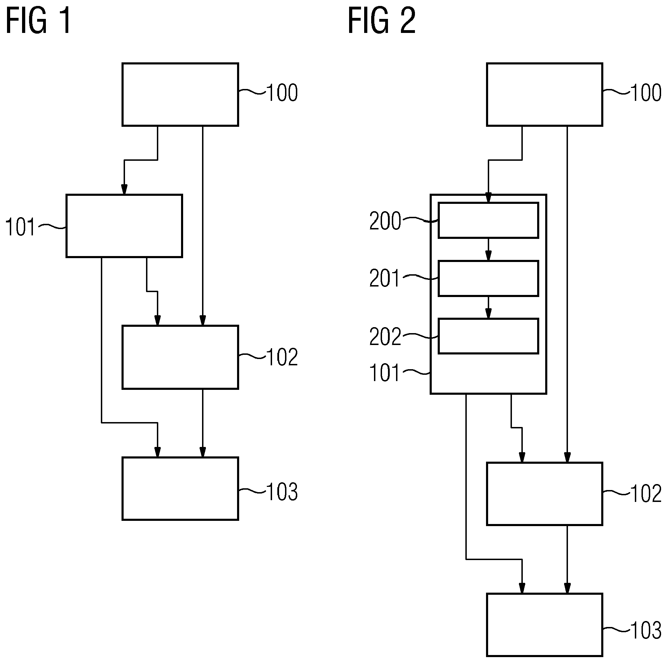

**Fig. 1** zeigt ein Verfahren für ein Berechnen eines personalisierten Patientenmodells in einem ersten Ausführungsbeispiel. Das personalisierte Patientenmodell weist ein externes Oberflächenmodell eines Patienten und ein Organmodell des Patienten auf. Das Verfahren für das Berechnen des personalisierten Patientenmodells weist die Verfahrensschritte 100 bis 103 auf.

Verfahrensschritt 100 kennzeichnet ein Erfassen von Metadaten des Patienten, wobei die Metadaten zumindest einer Metadatenkategorie zugeordnet sind.

Verfahrensschritt 101 kennzeichnet ein Ermitteln des externen Oberflächenmodells des Patienten und eines internen anatomischen Modells des Patienten, wobei das interne anatomische Modell eine Körperhöhle des Patienten aufweist, unter Verwendung der Metadaten des Patienten.

Verfahrensschritt 102 kennzeichnet ein Ermitteln des Organmodells des Patienten unter Verwendung der Metadaten des Patienten und des internen anatomischen Modells des Patienten.

Verfahrensschritt 103 kennzeichnet ein Berechnen des personalisierten Patientenmodells, wobei das externe Oberflächenmodell des Patienten und das Organmodell des Patienten kombiniert werden.

Die nachfolgende Beschreibung der folgenden Ausführungsbeispiele beschränkt sich im Wesentlichen auf die Unterschiede zu dem Ausführungsbeispiel in Fig. 1, wobei bezüglich gleich bleibender Verfahrensschritte auf die Beschreibung des Ausführungsbeispiels in Fig. 1 verwiesen wird. Im Wesentlichen gleich bleibende Verfahrensschritte sind grundsätzlich mit den gleichen Bezugszeichen beziffert.

**Fig. 2** zeigt das Verfahren für das Berechnen des personalisierten Patientenmodells in einem zweiten Ausführungsbeispiel. Die zumindest eine Metadatenkategorie weist in diesem Ausführungsbeispiel ein Gewicht des Patienten, eine Größe des Patienten und ein Geschlecht des Patienten auf.

Verfahrensschritt 200 kennzeichnet, dass beim Ermitteln des externen Oberflächenmodells des Patienten eine Pose des Patienten berücksichtigt wird.

Verfahrensschritt 201 kennzeichnet, dass ein externes Deformationsmodell auf physikalische Kräfte abgestimmt ist und wobei die physikalischen Kräfte die Pose des Patienten beeinflussen.

Verfahrensschritt 202 kennzeichnet, dass das Ermitteln des externen Oberflächenmodells des Patienten eine Verwendung des externen Deformationsmodells umfasst, wobei das externe Deformationsmodell auf die Pose des Patienten abgestimmt ist.

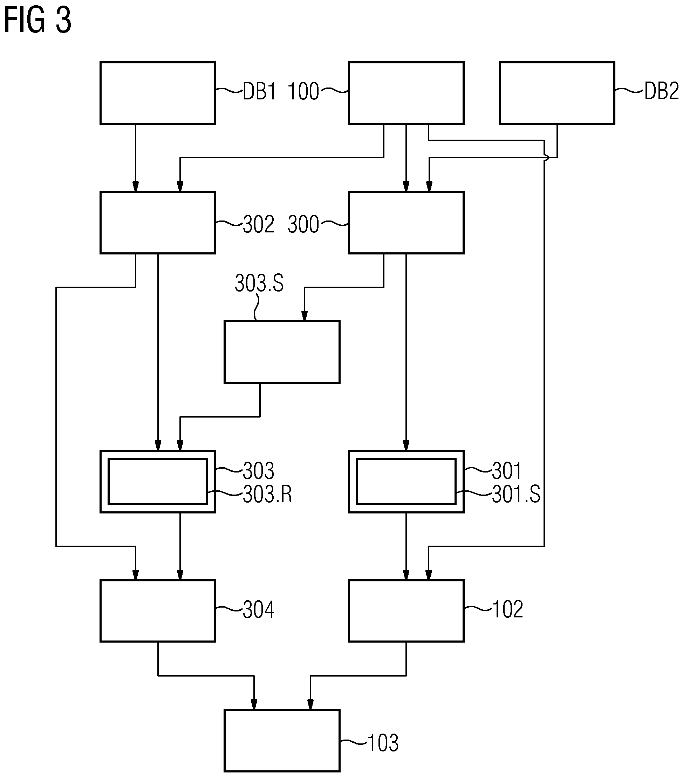

**Fig. 3** zeigt das Verfahren für das Berechnen des personalisierten Patientenmodells in einem dritten Ausführungsbeispiel.

Das Ermitteln des externen Oberflächenmodells des Patienten und des internen anatomischen Modells des Patienten erfolgt unter Verwendung einer Oberflächenmodelldatenbank DB1, welche externe Oberflächenmodelle aufweist, wobei jedem externem Oberflächenmodell oberflächenmodellbezogene Metadaten zugeordnet sind, und unter Verwendung einer medizinischen Bilddatenbank DB2, welche medizinische Bilddatensätze aufweist, wobei jedem medizinischen Bilddatensatz medizinische Metadaten zugeordnet sind, wobei die oberflächenmodellbezogenen Metadaten und die medizinischen Metadaten der zumindest einen Metadatenkategorie zugeordnet sind. Das Ermitteln des externen Oberflächenmodells des Patienten erfolgt in diesem Ausführungsbeispiel unter Verwendung der Oberflächenmodelldatenbank DB1 und der medizinischen Bilddatenbank DB2, wobei das Ermitteln des internen anatomischen Modells des Patienten ausschließlich unter Verwendung der medizinischen Bilddatenbank DB2 erfolgt.

Das Ermitteln des internen anatomischen Modells des Patienten umfasst folgende Verfahrensschritte 300, 301.S und 301.

Der Verfahrensschritt 300 kennzeichnet ein Auswählen zumindest eines medizinischen Bilddatensatzes aus den medizinischen Bilddatensätzen der medizinischen Bilddatenbank DB2 gemäß den Metadaten des Patienten.

Der Verfahrensschritt 301.S kennzeichnet, dass die Körperhöhle des internen anatomischen Modells des Patienten durch ein Segmentieren des zumindest einen medizinischen Bilddatensatzes gemäß einer zweiten Segmentierungsvorschrift ermittelt wird. Die zweite Segmentierungsvorschrift ist zu einem Segmentieren einer anatomischen Hülle oder einer konvexen Hülle, insbesondere der Körperhöhle, in dem zumindest einen medizinischen Bilddatensatz ausgebildet.

Der Verfahrensschritt 301 kennzeichnet ein Ermitteln der Körperhöhle des internen anatomischen Modells des Patienten unter Verwendung des zumindest einen medizinischen Bilddatensatzes.

Verfahrensschritt 302 kennzeichnet ein Auswählen eines abgeschätzten externen Oberflächenmodells aus den externen Oberflächenmodellen der Oberflächenmodelldatenbank DB1 gemäß den Metadaten des Patienten.

Verfahrensschritt 303 kennzeichnet ein Berechnen des externen Deformationsmodells unter Verwendung des abgeschätzten externen Oberflächenmodells, wobei das Berechnen des externen Deformationsmodells den Verfahrensschritt 300 und folgende Verfahrensschritte 303.S und 303.R umfasst.

Der Verfahrensschritt 303.S kennzeichnet, dass vor dem Registrieren des abgeschätzten externen Deformationsmodells der zumindest eine medizinischen Bilddatensatz gemäß einer ersten Segmentierungsvorschrift segmentiert wird. Die erste Segmentierungsvorschrift ist zu einem Segmentieren einer Oberfläche, insbesondere einer Haut, in dem zumindest einen medizinischen Bilddatensatz ausgebildet.

Der Verfahrensschritt 303.R kennzeichnet ein Registrieren des abgeschätzten externen Oberflächenmodells und des zumindest einen medizinischen Bilddatensatzes.

Verfahrensschritt 304 kennzeichnet ein Anwenden des externen Deformationsmodells auf das abgeschätzte externe Oberflächenmodell, wobei das externe Oberflächenmodell des Patienten erzeugt wird.

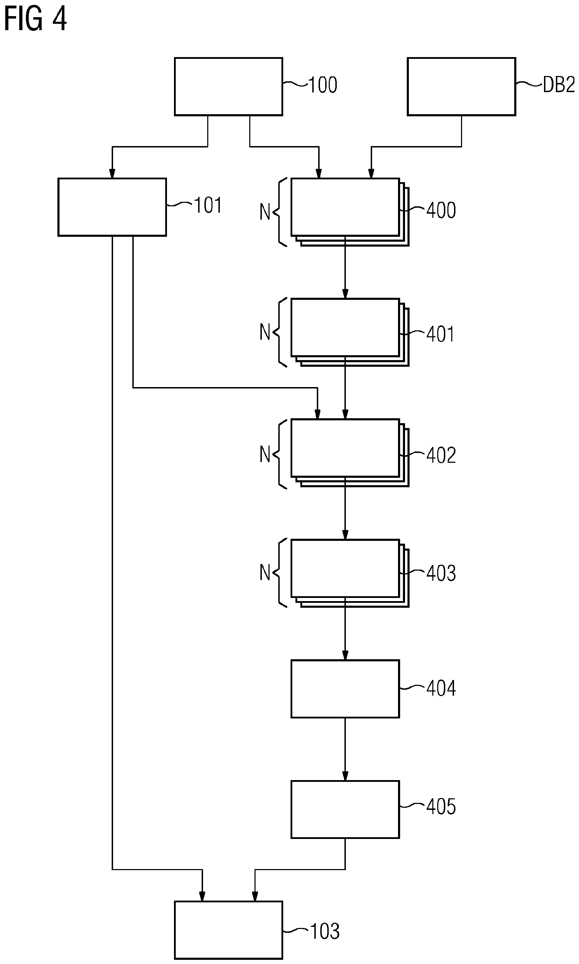

**Fig. 4** zeigt das Verfahren für das Berechnen des personalisierten Patientenmodells in einem vierten Ausführungsbeispiel.

Das Ermitteln des Organmodells des Patienten erfolgt unter Verwendung der medizinischen Bilddatenbank DB2, welche die medizinischen Bilddatensätze aufweist, wobei jedem medizinischen Bilddatensatz die medizinischen Metadaten zugeordnet sind, wobei die medizinischen Metadaten der zumindest einen Metadatenkategorie zugeordnet sind.

Das Ermitteln des Organmodells umfasst folgende Verfahrensschritte 400 bis 404.

Der Verfahrensschritt 400 kennzeichnet ein Auswählen zumindest eines medizinischen Bilddatensatzes aus den medizinischen Bilddatensätzen der medizinischen Bilddatenbank gemäß den Metadaten des Patienten. In diesem Ausführungsbeispiel werden eine Anzahl N größer 1 an medizinischen Bilddatensätzen ausgewählt. Dies ist insbesondere für das Klassifizieren des internen anatomischen Modells des Patienten vorteilhaft. Üblicherweise werden die N ähnlichsten medizinischen Bilddatensätze aus den medizinischen Bilddatensätzen der medizinischen Bilddatenbank ausgewählt.

Der Verfahrensschritt 401 kennzeichnet ein Segmentieren des zumindest einen medizinischen Bilddatensatzes gemäß einer dritten Segmentierungsvorschrift. Das Segmentieren wird in diesem Verfahrensschritt N-mal in den N ähnlichsten medizinischen Bilddatensätzen durchgeführt.

Der Verfahrensschritt 402 kennzeichnet ein Berechnen zumindest eines internen Deformationsmodells unter Verwendung des internen anatomischen Modells des Patienten und des segmentierten zumindest einen medizinischen Bilddatensatzes. In diesem Verfahrensschritt werden N interne Deformationsmodelle unter Verwendung des internen anatomischen Modells des Patienten und der N ähnlichsten medizinischen Bilddatensätzen separat berechnet.

Der Verfahrensschritt 403 kennzeichnet ein Anwenden des zumindest einen internen Deformationsmodells auf den segmentierten zumindest einen medizinischen Bilddatensatz. In diesem Verfahrensschritt werden die N internen Deformationsmodell auf die jeweiligen N ähnlichsten medizinischen Bilddatensätze angewendet.

Der Verfahrensschritt 404 kennzeichnet ein Klassifizieren des internen anatomischen Modells des Patienten gemäß dem deformierten zumindest einen medizinischen Bilddatensatz, wobei das Organmodell erzeugt wird. Das Klassifizieren erfolgt gemäß einer Mehrheitsentscheidung, ob ein untersuchtes Voxel ein Organ aufweist oder nicht. Üblicherweise ist das Klassifizieren umso robuster, desto mehr ähnliche medizinischen Bilddatensätze ausgewählt werden, sprich desto größer N ist. Entscheidend für das Klassifizieren ist insbesondere ein Maß für die Ähnlichkeit des zumindest einen medizinischen Bilddatensatzes. Das Maß für die Ähnlichkeit korreliert insbesondere mit einer Qualität des personalisierten Patientenmodells. Die Ähnlichkeit der medizinischen Bilddatensätze wird beispielsweise mittels einer Nächste-Nachbarn-Klassifikation (k-nearest-Neighbor) bestimmt. Vorteilsweise werden N medizinische Bilddatensätze mit einer hohen Ähnlichkeit ausgewählt.

Verfahrensschritt 405 kennzeichnet, dass das interne anatomische Modell zusätzlich zu der Körperhöhle eine weitere Körperhöhle aufweist, wobei die Körperhöhle einen Thorax des Patienten und die weitere Körperhöhle einen Abdomen des Patienten aufweist und wobei beim Ermitteln des Organmodells des Patienten ein Abstand zwischen der Körperhöhle Thorax und der weiteren Körperhöhle Abdomen minimiert wird.

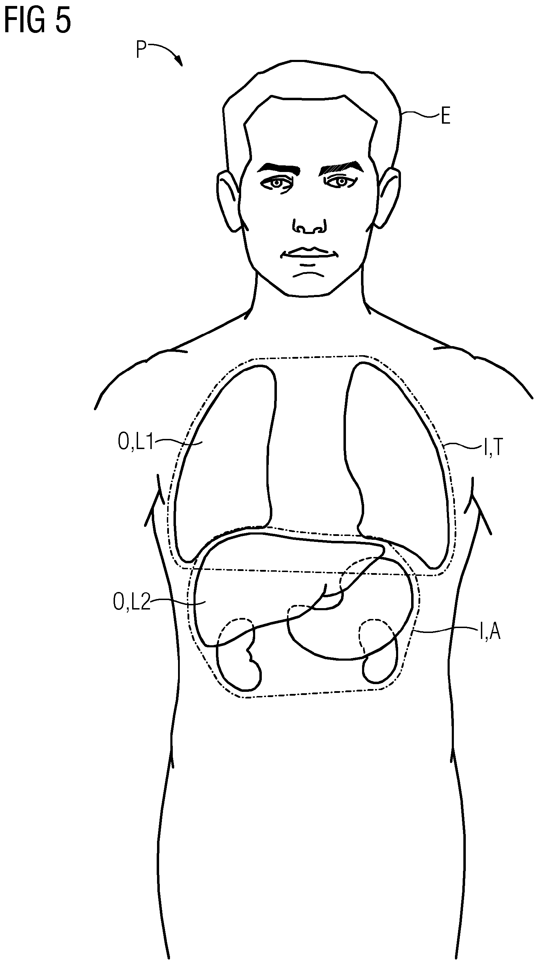

**Fig. 5** zeigt das personalisierte Patientenmodell in einer möglichen Ausführung. Fig. 5 zeigt insbesondere eine Abstraktion des personalisierten Patientenmodells. Neben der in Fig. 5 gezeigten Ausführung sind selbstverständlich auch weitere Ausführungen des personalisierten Patientenmodells denkbar. Das personalisierte Patientenmodell P weist das externe Oberflächenmodell E des Patienten, das interne anatomische Modell I des Patienten und das Organmodell O des Patienten auf. Das externe Oberflächenmodell E des Patienten zeigt einen Ausschnitt eines Oberkörpers des Patienten und grenzt die Haut des Patienten ab. Das interne anatomische Modell I des Patienten weist die Körperhöhle Thorax T und die weitere Körperhöhle Abdomen A auf. Das Organmodell O des Patienten ist innerhalb des internen anatomischen Modells I angeordnet. Das Organmodell O des Patienten weist mehrere Organe, in diesem Fall eine Lunge L1 des Patienten und eine Leber L2 des Patienten, auf. Die Körperhöhle Thorax T weist die Lunge L1 des Patienten auf und die weitere Körperhöhle Abdomen A weist die Leber L2 des Patienten auf. Eine Schnittmenge zwischen der Körperhöhle Thorax T und der weiteren Körperhöhle Abdomen A ist leer, obwohl in der Darstellung des personalisierten Patientenmodells P in Fig. 5 eine Überlagerung der Körperhöhle Thorax T und der weiteren Körperhöhle Abdomen A angedeutet wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Berechnen eines personalisierten Patientenmodells, welches ein externes Oberflächenmodell eines Patienten und ein Organmodell des Patienten aufweist, mit den folgenden Schritten:
- Erfassen von Metadaten des Patienten, wobei die Metadaten zumindest einer Metadatenkategorie zugeordnet sind,
- Ermitteln des externen Oberflächenmodells des Patienten und eines internen anatomischen Modells des Patienten, wobei das interne anatomische Modell eine Körperhöhle des Patienten aufweist, unter Verwendung der Metadaten des Patienten,
- Ermitteln des Organmodells des Patienten unter Verwendung der Metadaten des Patienten und des internen anatomischen Modells des Patienten und
- Berechnen des personalisierten Patientenmodells, wobei das externe Oberflächenmodell des Patienten und das Organmodell des Patienten kombiniert werden.

2. Verfahren nach Anspruch 1, wobei die zumindest eine Metadatenkategorie mindestens einen Eintrag der folgenden Liste aufweist:
- ein Gewicht des Patienten,
- eine Größe des Patienten,
- ein Geschlecht des Patienten.

3. Verfahren nach einem der vorhergehenden Ansprüche, wobei beim Ermitteln des externen Oberflächenmodells des Patienten eine Pose des Patienten berücksichtigt wird.

4. Verfahren nach Anspruch 3, wobei das Ermitteln des externen Oberflächenmodells des Patienten eine Verwendung eines externen Deformationsmodells umfasst, wobei das externe Deformationsmodell auf die Pose des Patienten abgestimmt ist.

5. Verfahren nach Anspruch 4, wobei das externe Deformationsmodell auf physikalische Kräfte abgestimmt ist und wobei die physikalischen Kräfte die Pose des Patienten beeinflussen.

6. Verfahren nach einem der vorhergehenden Ansprüche, wobei das Ermitteln des externen Oberflächenmodells des Patienten und des internen anatomischen Modells des Patienten unter Verwendung einer Oberflächenmodelldatenbank, welche externe Oberflächenmodelle aufweist, wobei jedem externem Oberflächenmodell oberflächenmodellbezogene Metadaten zugeordnet sind, und unter Verwendung einer medizinischen Bilddatenbank, welche medizinische Bilddatensätze aufweist, wobei jedem medizinischen Bilddatensatz medizinische Metadaten zugeordnet sind, erfolgt, wobei die oberflächenmodellbezogenen Metadaten und die medizinischen Metadaten der zumindest einen Metadatenkategorie zugeordnet sind.

7. Verfahren nach Anspruch 6, wobei das Ermitteln des externen Oberflächenmodells des Patienten unter Verwendung der Oberflächenmodelldatenbank und der medizinischen Bilddatenbank erfolgt und wobei das Ermitteln des internen anatomischen Modells des Patienten ausschließlich unter Verwendung der medizinischen Bilddatenbank erfolgt.

8. Verfahren nach einem der vorhergehenden Ansprüche, wobei das Ermitteln des internen anatomischen Modells des Patienten unter Verwendung einer medizinischen Bilddatenbank erfolgt, welche medizinische Bilddatensätze aufweist, wobei jedem medizinischen Bilddatensatz medizinische Metadaten zugeordnet sind, wobei die medizinischen Metadaten der zumindest einen Metadatenkategorie zugeordnet sind, und folgende Schritte umfasst:
- Auswählen zumindest eines medizinischen Bilddatensatzes aus den medizinischen Bilddatensätzen der medizinischen Bilddatenbank gemäß den Metadaten des Patienten und
- Ermitteln der Körperhöhle des internen anatomischen Modells des Patienten unter Verwendung des zumindest einen medizinischen Bilddatensatzes.

9. Verfahren nach einem der vorhergehenden Ansprüche, wobei das Ermitteln des externen Oberflächenmodells des Patienten unter Verwendung einer Oberflächenmodelldatenbank erfolgt, welche externe Oberflächenmodelle aufweist, wobei jedem externem Oberflächenmodell oberflächenmodellbezogene Metadaten zugeordnet sind, wobei die oberflächenmodellbezogenen Metadaten der zumindest einen Metadatenkategorie zugeordnet sind, und folgende Schritte umfasst:
- Auswählen eines abgeschätzten externen Oberflächenmodells aus den externen Oberflächenmodellen der Oberflächenmodelldatenbank gemäß den Metadaten des Patienten,
- Berechnen eines externen Deformationsmodells unter Verwendung des abgeschätzten externen Oberflächenmodells und
- Anwenden des externen Deformationsmodells auf das abgeschätzte externe Oberflächenmodell, wobei das externe Oberflächenmodell des Patienten erzeugt wird.

10. Verfahren nach Anspruch 9, wobei das Berechnen des externen Deformationsmodells unter Verwendung einer medizinischen Bilddatenbank erfolgt, welche medizinische Bilddatensätze aufweist, wobei jedem medizinischen Bilddatensatz medizinische Metadaten zugeordnet sind, wobei die medizinischen Metadaten der zumindest einen Metadatenkategorie zugeordnet sind, und folgende Schritte umfasst:
- Auswählen zumindest eines medizinischen Bilddatensatzes aus den medizinischen Bilddatensätzen der medizinischen Bilddatenbank gemäß den Metadaten des Patienten und
- Registrieren des abgeschätzten externen Oberflächenmodells und des zumindest einen medizinischen Bilddatensatzes.

11. Verfahren nach den Ansprüchen 8 und 10,
- wobei vor dem Registrieren des abgeschätzten externen Deformationsmodells der zumindest eine medizinischen Bilddatensatz gemäß einer ersten Segmentierungsvorschrift segmentiert wird, und/oder
- wobei die Körperhöhle des internen anatomischen Modells des Patienten durch ein Segmentieren des zumindest einen medizinischen Bilddatensatzes gemäß einer zweiten Segmentierungsvorschrift ermittelt wird.

12. Verfahren nach einem der vorhergehenden Ansprüche, wobei das interne anatomische Modell zusätzlich zu der Körperhöhle eine weitere Körperhöhle aufweist, wobei die Körperhöhle einen Thorax des Patienten und die weitere Körperhöhle einen Abdomen des Patienten aufweist und wobei beim Ermitteln des Organmodells des Patienten ein Abstand zwischen der Körperhöhle Thorax und der weiteren Körperhöhle Abdomen minimiert wird.

13. Verfahren nach einem der vorhergehenden Ansprüche, wobei das Ermitteln des Organmodells des Patienten unter Verwendung einer medizinischen Bilddatenbank, welche medizinische Bilddatensätze aufweist, wobei jedem medizinischen Bilddatensatz medizinische Metadaten zugeordnet sind, erfolgt, wobei die medizinischen Metadaten der zumindest einen Metadatenkategorie zugeordnet sind.

14. Verfahren nach Anspruch 13, wobei das Ermitteln des Organmodells folgende Schritte umfasst:
- Auswählen zumindest eines medizinischen Bilddatensatzes aus den medizinischen Bilddatensätzen der medizinischen Bilddatenbank gemäß den Metadaten des Patienten,
- Segmentieren des zumindest einen medizinischen Bilddatensatzes gemäß einer dritten Segmentierungsvorschrift,
- Berechnen zumindest eines internen Deformationsmodells unter Verwendung des internen anatomischen Modells des Patienten und des segmentierten zumindest einen medizinischen Bilddatensatzes,
- Anwenden des zumindest einen internen Deformationsmodells auf den segmentierten zumindest einen medizinischen Bilddatensatz und
- Klassifizieren des internen anatomischen Modells des Patienten gemäß dem deformierten zumindest einen medizinischen Bilddatensatz, wobei das Organmodell erzeugt wird.

15. Recheneinheit für ein Berechnen eines personalisierten Patientenmodells, aufweisend ein Rechenmodul, wobei die Recheneinheit zu einem Ausführen eines Verfahrens nach einem der Ansprüche 1 bis 14 ausgebildet ist.

16. Computerprogrammprodukt, welches direkt in einen Speicher einer programmierbaren Recheneinheit ladbar ist, mit Programmcodemitteln, um ein Verfahren nach einem der Ansprüche 1 bis 14 auszuführen, wenn das Computerprogrammprodukt in der Recheneinheit ausgeführt wird.

## Geänderte Patentansprüche

### Geänderte Patentansprüche gemäss Regel 137(2) EPÜ.

1. Verfahren für ein Berechnen eines personalisierten Patientenmodells, welches ein externes Oberflächenmodell eines Patienten und ein Organmodell des Patienten aufweist, mit den folgenden Schritten:
- Erfassen von Metadaten des Patienten, wobei die Metadaten zumindest einer Metadatenkategorie zugeordnet sind,
- Ermitteln des externen Oberflächenmodells des Patienten und eines internen anatomischen Modells des Patienten, wobei das interne anatomische Modell eine Körperhöhle des Patienten aufweist, unter Verwendung der Metadaten des Patienten, wobei das Ermitteln des externen Oberflächenmodells des Patienten und des internen anatomischen Modells des Patienten unter Verwendung einer Oberflächenmodelldatenbank, welche externe Oberflächenmodelle aufweist, wobei jedem externem Oberflächenmodell oberflächenmodellbezogene Metadaten zugeordnet sind, und unter Verwendung einer medizinischen Bilddatenbank, welche medizinische Bilddatensätze aufweist, wobei jedem medizinischen Bilddatensatz medizinische Metadaten zugeordnet sind, erfolgt, wobei die oberflächenmodellbezogenen Metadaten und die medizinischen Metadaten der zumindest einen Metadatenkategorie zugeordnet sind,
- Ermitteln des Organmodells des Patienten unter Verwendung der Metadaten des Patienten und des internen anatomischen Modells des Patienten, wobei das Ermitteln des Organmodells des Patienten unter Verwendung der medizinischen Bilddatenbank erfolgt,
- Berechnen des personalisierten Patientenmodells, wobei das externe Oberflächenmodell des Patienten und das Organmodell des Patienten kombiniert werden und
- Bereitstellen des personalisierten Patientenmodells für einen Anwendungsfall,
wobei eine Anwendung des personalisierten Patientenmodells in zumindest einem der folgenden Anwendungsfälle erfolgt:
- eine Positionierung des Patienten gemäß dem personalisierten Patientenmodell für eine bildgebende Untersuchung oder eine Therapiesitzung,
- eine Kollisionsvermeidung von einem medizinischen Bildgebungsgerät oder Therapiegerät mit dem Patienten gemäß Informationen des personalisierten Patientenmodells,
- eine Berechnung einer Dosisverteilung gemäß dem personalisierten Patientenmodell während der bildgebenden Untersuchung oder der Therapiesitzung,
- eine Berechnung einer Dosisverteilung in einem Untersuchungsraum gemäß dem personalisierten Patientenmodell,
- eine automatische Positionierung des medizinischen Bildgebungsgeräts oder des Therapiegeräts gemäß dem personalisierten Patientenmodell,
- ein Optimieren von einem Messparameter des medizinischen Bildgebungsgeräts gemäß dem personalisierten Patientenmodell in Hinblick auf eine optimale Dosis und/oder Bildqualität.

2. Verfahren nach Anspruch 1, wobei die zumindest eine Metadatenkategorie mindestens einen Eintrag der folgenden Liste aufweist:
- ein Gewicht des Patienten,
- eine Größe des Patienten,
- ein Geschlecht des Patienten.

3. Verfahren nach einem der vorhergehenden Ansprüche, wobei beim Ermitteln des externen Oberflächenmodells des Patienten eine Pose des Patienten berücksichtigt wird.

4. Verfahren nach Anspruch 3, wobei das Ermitteln des externen Oberflächenmodells des Patienten eine Verwendung eines externen Deformationsmodells umfasst, wobei das externe Deformationsmodell auf die Pose des Patienten abgestimmt ist.

5. Verfahren nach Anspruch 4, wobei das externe Deformationsmodell auf physikalische Kräfte abgestimmt ist und wobei die physikalischen Kräfte die Pose des Patienten beeinflussen.

6. Verfahren nach einem der vorhergehenden Ansprüche, wobei das Ermitteln des externen Oberflächenmodells des Patienten unter Verwendung der Oberflächenmodelldatenbank und der medizinischen Bilddatenbank erfolgt und wobei das Ermitteln des internen anatomischen Modells des Patienten ausschließlich unter Verwendung der medizinischen Bilddatenbank erfolgt.

7. Verfahren nach einem der vorhergehenden Ansprüche, wobei das Ermitteln des internen anatomischen Modells des Patienten unter Verwendung der medizinischen Bilddatenbank erfolgt und folgende Schritte umfasst:
- Auswählen zumindest eines medizinischen Bilddatensatzes aus den medizinischen Bilddatensätzen der medizinischen Bilddatenbank gemäß den Metadaten des Patienten und
- Ermitteln der Körperhöhle des internen anatomischen Modells des Patienten unter Verwendung des zumindest einen medizinischen Bilddatensatzes, wobei die Körperhöhle des internen anatomischen Modells des Patienten durch ein Segmentieren des zumindest einen medizinischen Bilddatensatzes gemäß einer zweiten Segmentierungsvorschrift ermittelt wird.

8. Verfahren nach einem der vorhergehenden Ansprüche, wobei das Ermitteln des externen Oberflächenmodells des Patienten unter Verwendung der Oberflächenmodelldatenbank erfolgt und folgende Schritte umfasst:
- Auswählen eines abgeschätzten externen Oberflächenmodells aus den externen Oberflächenmodellen der Oberflächenmodelldatenbank gemäß den Metadaten des Patienten,
- Berechnen des externen Deformationsmodells unter Verwendung des abgeschätzten externen Oberflächenmodells und
- Anwenden des externen Deformationsmodells auf das abgeschätzte externe Oberflächenmodell, wobei das externe Oberflächenmodell des Patienten erzeugt wird.

9. Verfahren nach Anspruch 8, wobei das Berechnen des externen Deformationsmodells unter Verwendung der medizinischen Bilddatenbank erfolgt und folgende Schritte umfasst:
- Auswählen zumindest eines medizinischen Bilddatensatzes aus den medizinischen Bilddatensätzen der medizinischen Bilddatenbank gemäß den Metadaten des Patienten und
- Registrieren des abgeschätzten externen Oberflächenmodells und des zumindest einen medizinischen Bilddatensatzes, wobei vor dem Registrieren des abgeschätzten externen Deformationsmodells der zumindest eine medizinischen Bilddatensatz gemäß einer ersten Segmentierungsvorschrift segmentiert wird.

10. Verfahren nach einem der vorhergehenden Ansprüche, wobei das interne anatomische Modell zusätzlich zu der Körperhöhle eine weitere Körperhöhle aufweist, wobei die Körperhöhle einen Thorax des Patienten und die weitere Körperhöhle einen Abdomen des Patienten aufweist und wobei beim Ermitteln des Organmodells des Patienten ein Abstand zwischen der Körperhöhle Thorax und der weiteren Körperhöhle Abdomen minimiert wird.

11. Verfahren nach einem der vorhergehenden Ansprüche, wobei das Ermitteln des Organmodells folgende Schritte umfasst:
- Auswählen zumindest eines medizinischen Bilddatensatzes aus den medizinischen Bilddatensätzen der medizinischen Bilddatenbank gemäß den Metadaten des Patienten,
- Segmentieren des zumindest einen medizinischen Bilddatensatzes gemäß einer dritten Segmentierungsvorschrift,
- Berechnen zumindest eines internen Deformationsmodells unter Verwendung des internen anatomischen Modells des Patienten und des segmentierten zumindest einen medizinischen Bilddatensatzes,
- Anwenden des zumindest einen internen Deformationsmodells auf den segmentierten zumindest einen medizinischen Bilddatensatz und
- Klassifizieren des internen anatomischen Modells des Patienten gemäß dem deformierten zumindest einen medizinischen Bilddatensatz, wobei das Organmodell erzeugt wird.

12. Recheneinheit für ein Berechnen eines personalisierten Patientenmodells, aufweisend ein Rechenmodul, wobei die Recheneinheit zu einem Ausführen eines Verfahrens nach einem der Ansprüche 1 bis 11 ausgebildet ist.

13. Computerprogrammprodukt, welches direkt in einen Speicher einer programmierbaren Recheneinheit ladbar ist, mit Programmcodemitteln, um ein Verfahren nach einem der Ansprüche 1 bis 11 auszuführen, wenn das Computerprogrammprodukt in der Recheneinheit ausgeführt wird.
